# EUROPEAN PATENT APPLICATION

(11) **EP 1 803 358 A1**
(43) Date of publication of application: **04.07.2007**
(21) Application number: 05113022.7
(22) Date of filing: 28.12.2005
(51) Int. Cl.: A23L 1/29, A61P 37/04, A23L 1/308, A23L 1/305

(54) **Infant immunological formula**

(71) Applicant: LABORATORIOS ORDESA, S.L, 08830 Sant Boi de Llobregat, Barcelona (ES)
(72) Inventor: Rivero Urgell, Montserrat, 08830, SANT BOI DEL LLOBREGAT (ES); Chifré Petit, Ricard, 08830, SANT BOI DEL LLOBREGAT (ES); Rodriguez-Palmero, María, 08830, SANT BOI DEL LLOBREGAT (ES); Campoy Folgoso , Cristina, 18012, GRANADA (ES)
(74) Representative: Barlocci, Anna

(57) **Abstract**

The invention relates to nutritional compositions and infant formulas comprising a combination of colostrum or a colostrum-derived product and a prebiotic component. The composition of the invention provides an improvement of the regulation and stimulation of the development of the immune system.

## Description

The present invention relates to improved infant formulas. More specifically, the invention relates to infant formulas which contain colostrum or a colostrum-derived product and a prebiotic component selected from the group consisting of galactooligosaccharides, inulin, and a mixture thereof.

### BACKGROUND ART

The composition of human milk serves as a valuable reference for improving nutritional formulas. It is known that in addition to essential nutrients, human milk contains several types of bioactive factors, including growth factors, enzymes, antimicrobial factors, antiinflammatory agents, immunoglobulins, oligosaccharides, transporters and peptide and non-peptide hormones. Many of these factors appear in high concentrations in colostrum and can play multiple functional roles.

Nevertheless, many constituents in human milk are bioactive and show synergies between themselves. An objective looked for by infant formulas is to manage to achieve that the activity of the different components present in the infant formula is similar to the activity of these in the human milk

There is currently a great interest in the role of prebiotics as promoters of bifidogenic flora in children, since they provide nutrients for all colonic bacteria, therefore contributing to a beneficial increase in the pH and bacterial flora.

EP 1125507 relates to novel inulin products and compositions thereof, which consists of a mixture of an easily fermentable inulin component and a hardly fermentable inulin component in a specific weight ratio, which modulate the bacterial flora composition in the large intestine of humans and mammals and which modulate the fermentation pattern of inulin in humans and mammals.

EP 303201 relates to a method of producing a processed milk containing galactooligosaccharides comprising incubating an animal milk with a β-galactosidase.

WO 02/47612 relates to dietary supplement compositions containing effective amounts of colostrum, lactoferrin, pectin and β-glucan, for promotion and maintenance of immune system.

Chin Med J 2004, 117(6): 927-931 relates to milk formula supplementation with galactooligosaccharides (GOS) to improve intestinal microflora and fermentation in term infants.

The WO 99/64022 application relates to nutritive compositions comprising whey, selenium, fructooligosaccharides (FOS), and colostrum for the creation and maintenance of a healthy intestinal flora and for the enhancement of the immune system.

The WO 05/003329 application relates to novel strains of *Bifidobacterium bifidum* that produce a novel galactosidase enzyme activity capable of converting lactose to a novel mixture of galactooligosaccharides. It relates to the use of a *Bifidobacterial* strain to produce a novel galactooligosaccharide composition that is capable of promoting the growth of *Bifidobacteria* in the gut. It also relates to a novel composition of the galactooligosaccharide products.

Moro et al. (J Pediatr Gastroenterol Nutr 2002; 34:291-295) have tested a synergistic mixture of neutral galactooligosaccharides and long-chain fructooligosaccharides. After 28 days of feeding, the term infants fed formula supplemented with the GOS/FOS mixture, at concentration of 0.4 g/100 mL or 0.8 g/100 mL, respectively, exhibited a dose-dependent stimulating effect on the growth of *Bifidobacteria* and lactobacilli in the intestine. This combination resulted also in an increase of stool frequency and a reduction of stool consistency, closer to reference breast-fed infants.

EP 1237419B1 relates to a infant formula which comprises a protein component having a phosphorus content of less than 0.75 g P/100 g protein; and a lipid component that can be easily digested by an infant, comprising fatty acid triglycerides, in which palmitic acid residues make up more than 10 % (w/w) of all fatty acid residues present in the triglycerides, at least 30 % of the palmitic acid residues in the triglycerides being in the Sn2 position of the triglycerides. The infant formula may also comprises a prebiotic component, e.g. galactooligosaccharides. According to this document, the problem to be solve by the infant formula described therein is to overcome the undesired disturbances in the natural gut flora and/or to an unnatural gut flora, which is not resolved by conventional formulas.

Thus, there is a need for new alternative infant formulas addressed to provide to the infant or young child an improvement of the regulation and stimulation of the development of the immune system. The improvement of the immune system is desirable not only for infants of during the first four to six months of life but also by infants aged over four months.

### SUMMARY OF THE INVENTION

The present invention relates to nutritional compositions comprising a combination of colostrum or a colostrum-derived product and a prebiotic component selected from the group consisting of galactooligosaccharides, inulin, and mixtures thereof. The nutritional composition of the invention provides an improvement of the regulation and stimulation of the development of the immune system, as demonstrated by the determination and analysis of some biochemical parameters in connection with the regulation and stimulation of the development of the immune system. When administered in combination, the effects of colostrum or a colostrum-derived product plus the prebiotic component above mentioned over the immune system are surprisingly beneficial due to an unexpected synergistic effect. The nutritional composition of the invention may be used in the preparation of an infant immunological formula.

Therefore, a first aspect of the invention relates to a nutritional composition comprising colostrum or a colostrum-derived product, and a prebiotic component selected from the group consisting of galactooligosaccharides, inulin, and mixtures thereof, which provides an improvement of the regulation and stimulation of the development of the immune system.

A second aspect of the invention relates to infant formulas which provides an improvement of the regulation and stimulation of the development of the immune system, which comprises the nutritional compositions of the invention. A third aspect of the invention relates to the use of such infant formula to regulate and stimulate the development of the immune system.

This third aspect may alternatively be formulated as a method for regulate and stimulate the development of the immune system and mammals through the administration of an effective amount of the nutritive compositions as described herein.

These and other objects of the present invention will be further described in the detailed description section that follows.

### DETAILED DESCRIPTION OF THE INVENTION

For the purposes of the present invention, the term "infant formula" relates to foodstuffs intended for particular nutritional use by infants during the first four to six months of life and satisfying by themselves the nutritional requirements of this category of persons, also for nutritional use by premature babies, as well as by infants aged over four months and constituting the principal liquid element in a progressively diversified diet of this category of persons (also so called "follow-on formulas" as known by those skilled in the art). Therefore, as defined above, the term "infant formula", as used in the present invention, relates to pre-term, term and follow-on formulas as known by the skilled person in the art.

The term "nutritional composition" as used herein refers to a composition designed for infants, toddlers, children, adults, or combinations thereof, which preferably contains sufficient protein, carbohydrate, lipid, vitamins, minerals, and electrolytes to potentially serve as the sole source of nutrition when provided in sufficient quantity.

It has to be understood that infants can be fed solely with infant formulas, or that the infant formula can be used as a complement of human milk.

Colostrum is a thin milky fluid secreted by the mammary gland of mammals 24-36h after parturition. It is a unique combination of beneficial nutrients including protective antibodies, fats, carbohydrates, vitamins and minerals. The immunological components of colostrum include IgG, IgM and IgA. These components confer passive immunity to the neonate and protection against infection during the initial period after parturition.

Colostrum supplies several types of growth-promoting factors; some of them are epidermal growth factor that induces synthesis of epidermal cells, transforming growth factor that induces collagen synthesis, wound healing and bone-resorption; and insulin-like growth factor (IGF-1) which induces epithelial cell proliferation and is necessary for growth and repairment of healthy tissue. Colostrum also contains proline-rich protein (PRP) that induces growth and differentiation of resting B lymphocytes, which is essential for the development of immune response. B lymphocytes are referred to as "resting" during their inactive phase, i.e., while not responding to stimulation by an antigen. In addition, PRP is understood to aid in the maturation of thymocytes, originated from the thymus, a crucial organ for the early development of the immune system. PRP is further understood to aid in the generation of T-suppressor and T-helper cells, demonstrating that PRP can have either a stimulating or a suppressing effect on immune response. [K. Staroscik et al., Immunol., 20 (12) :1277-82 (1983)].

Therefore, one of the biological activities of colostrum is related to activation and regulation of the immune system. Human colostrums has been shown to activate the immune system by stimulating secretion of cytokines such as IL-1, IL-3, and IL-6 by peripheral blood mononuclear cell *in vitro,* in such a way that this activation is important when the immune system is needed.

The infant formula of the invention may comprise colostrum or a colostrum-derived product. The colostrum to be used according to the present invention is not limiting. It is preferably derived from bovine animals. However, the animal or origin is not limiting and other sources of colostrum include ovine or caprine sources. The colostrum or colostrum-derived product may be in any form, such as liquid, powdered and/or freeze-dried.

The term "colostrum-derived product" as intended in the present invention relates to fractions of colostrum resulting of remove some content of colostrum, or resulting from modifying the composition of the colostrum by enzymatic or chemical treatment of the colostrum.

According to a preferred embodiment, the colostrum-derived product used is a fraction of colostrum enriched in whey proteins. According to a more preferred embodiment, the colostrum-derived product comprises between 75-90 g /100 g of whey protein.

According to a preferred embodiment of the invention, the infant formula presents a total protein content with a ratio of whey protein to casein between 50:50 to 80:20. In another preferred embodiment, the whey protein to casein ratio is of 60:40, even more preferred of 70:30.

The amount of colostrum or colostrum-derived product in the formula may vary over a wide range; preferably the infant formula comprises up to 1.37 g/100 ml of colostrum or a colostrum-derived product.

In a preferred embodiment, the colostrum-derived product used in the infant formula of the invention is prepared through the enzymatic hydrolysis of the casein proteins presents in milk, following of separation and elimination of the casein proteins and fats; afterwards the resulting product is undergone to microfiltration and finally is freeze-dried.

On another hand, an approach to dietary manipulation of the gut microflora is the use of a prebiotic, which is defined as a non-digestible food ingredient that beneficially affects the host by selectively stimulating the growth and/or activity of one or a limited number of bacteria in the colon, thereby resulting in an improvement in the health of the host.

One of the components that can be used as prebiotics are Galactooligosaccharides (GOS) are generally represented by a general formula Gal-(Gal)n-Glc, wherein Gal represents a galactose residue, Glc represents a glucose residue and n represents an integer representing the number of galactose units linked to the terminal glucose unit. The number of saccharide units (galactose and glucose units) in one molecule, i. e. the values n+2 in the above formula, are commonly referred to as the degree of polymerisation (DP).

GOS are one of major components of breast milk oligosaccharide, and is known also as a proliferation accelerating factor of bifidobacterium which is one of useful enterobacteria in human intestines. Galactose is a major component of some very important brain lipids including myelin and it has been suggested that galactose derived oligosaccharides may play a role in neonatal brain development (Kunz C. et. al. 1999, Br J Nutr; 82: 391-399).

The GOS can be produced from lactose by the transglycosylating activity of beta-galactosidase. Beta-galacto-oligosaccharides consist of a number of beta-1,6-linked galactosyl residues linked to a terminal glucose unit via an beta-1,4-bond. Another type of galactooligosaccharides is isolated from soybeans. These alpha-galacto-oligosaccharides (galactosyl-sucrose oligosaccharides) include raffinose, stachyose and verbascose and consist of galactose residues linked alpha-1,6 to the glucose moiety of sucrose. The physiological effects of these oligosaccharides appear to be similar to the beta-linked galactose oligomers. The composition of galactooligosaccharides may vary in chain length and in type of linkage between the monomer units.

The combination, according to the present invention, of colostrum or a colostrum-derived product plus the galactooligosaccharide component above mentioned, has a surprisingly beneficial effect over the immune system due to an unexpected synergistic effect. Therefore, according to a preferred embodiment of the present invention, the prebiotic component is a galactooligosaccharide.

Inulin is a dietary fiber that is composed of a mixture of oligomers and polymers of fructose. Inulin is a carbohydrate which occurs in many plants and which can be produced by certain bacteria. Inulin from plant origin consists of a polydisperse composition of chains of oligo-and polysaccharides which are composed of fructose units linked to each other through β(2-1) fructosyl-fructose linkages, and which mostly terminate in one glucose unit. Inulin from plant origin is usually composed of linear chains, but may also contain some branched chains. Inulin can be generally represented, depending from the terminal carbohydrate unit, by the general formula GFn and Fm, wherein G represents a glucose unit, F represents a fructose unit, n is an integer representing the number of fructose units linked to the terminal glucose unit, and m is an integer representing the number of fructose units linked to each other in the carbohydrate chain. The number of saccharide units (fructose and glucose units) in one molecule, i. e. the values n+1 and m in the above formula, are commonly referred to as the degree of polymerisation (DP).

Inulin which is a natural dietary fiber, has shown to have desirable health effects on humans and many vertebrate animals, such as e. g. beneficial prebiotic effects, in view of which inulin is already widely used in foods and drinks to improve the functioning of the digestive system, including stimulation of the grow, metabolism and activity of *Bifidobacteria* while reducing those of less desirable intestinal bacteria. Inulin has also been disclosed to have beneficial effects on lipid metabolism and to have prophylactic and therapeutic effects, e. g. on the genesis and development of breast cancer (EP 692252) and of colon cancer in humans and non-bovine mammals (EP 879600).

Inulin molecules with a low degree of polymerisation, usually defined as a (DP) < 10, are termed herein short-chain inulin. Inulin with a (DP) ≥ 20 is termed herein long-chain inulin whereas inulin with a (DP) from 10 to < 20 is termed herein medium-chain inulin. Unless otherwise specified, the term inulin used herein refers to linear as well as branched inulin, and includes inulin molecules with a (DP)<20 as well as inulin molecules with a (DP)≥20.

According to one embodiment of the present invention, the inulin product of the invention consists of a mixture of an easily fermentable inulin component and a hardly fermentable inulin component in a specific weight ratio ranging from 10/90 to 70/30. In a preferred inulin product of the invention, said weight ratio is preferably ranging from 20/80 to 65/35, more preferably from 35/65 to 65/35, and even more preferably from 40/60 to 45/55, typically about 50/50.

By easily fermentable inulin is meant herein linear as well as branched inulin-type products which are completely or almost completely fermented in the proximal part (the ascendent part) of the large intestine of humans and mammals. Typical easily fermentable inulin are short-chain inulin (i.e. inulin with a (DP) < 10) and agave inulin (i.e. a branched inulin). By hardly fermentable inulin is meant herein linear as well as branched inulin-type products of which the fermentation hardly starts in the proximal part of the large intestine and which are mainly fermented, though at a low rate, in the distal part (the transversal part and/or the descendent part) of the large intestine of humans and mammals. Typical hardly fermentable inulin are long-chain inulin, i.e. linear as well as branched inulin with a (DP)≥ 20, and inulin in a particular crystallographic form or a particular physical appearance form which does not enable easy and significant fermentation in the proximal part of the large intestine of humans and mammals.

Inulin is used as a source of energy in the intestinal tract mainly by bacteria of the genus *Bifidobacterium.* When inulin is administered in the diet, the *Bifidobacteria* increase significantly, becoming the predominant bacteria in the intestinal population, and the *Clostridia,* which are a measure of potentially pathogenic microorganisms, are significantly reduced. *Bifidobacteria* are human intestinal bacteria that provide beneficial effects on gastrointestinal health. Other important groups of bacteria in the mixed population in the intestines, such as *Fusobacteria, Lactobacillus,* and aerobic bacteria, are not significantly affected by the administration of inulin.

The inulin product of the invention has the potential to quickly and significantly modify and modulate the composition of the bacterial flora in the large intestine, in the proximal part as well as in the distal part of the large intestine.

Furthermore, the combination, according to the present invention, of colostrum or a colostrum-derived product plus the inulin component above mentioned, has a surprisingly beneficial effect over the immune system due to an unexpected synergistic effect. Accordingly, a preferred embodiment of the invention relates to a infant formula comprising inulin as prebiotic component. Particularly preferred are those formulas wherein the prebiotic component consists of a mixture of an easily fermentable inulin component and a hardly fermentable inulin as described above.

The amount of the one or more prebiotic components used will generally depend upon the specific prebiotic component used, as well as on the amount of formula to be fed to the infant per day, e.g. as one or more, and preferably four or more, feedings per day. In a preferred embodiment of the invention, the infant formula comprises up to 1.2 g/100 ml of the prebiotic component; more preferably, the infant formula comprises up to 0.8 g/100 ml of the prebiotic component.

According to a preferred embodiment of the invention, the infant formula comprises also other components which are involved in the improvement of the regulation and stimulation of the development of the immune system.

Fatty acids are carboxylic acids and are classified based on the length and on saturation characteristics of the carbon chain. Short chain fatty acids have from 2 to about 6 carbons and are typically saturated. Medium chain fatty acids have from about 6 to about 14 carbons and are also typically saturated. Long chain fatty acids have from 16 to 24 or more carbons and may also be saturated or unsaturated. In longer fatty acids there may be one or more points of instauration, giving rise to the terms "monounsaturated" and "polyunsaturated", respectively. Long chain polyunsaturated fatty acids, (LC-PUFAs) having 20 or more carbons are of particular interest in the present invention.

LC-PUFAs are categorized according to the number and position of double bonds in the fatty acids according to a nomenclature well understood by the biochemist. There are two series or families of LC-PUFAs, depending on the position of the double bond closest to the methyl end of the fatty acid: the n-3 serie contain a double bond at the third carbon, while the n-6 serie have no double bond until the sixth carbon. Thus, arachidonic acid (AA) has a chain length of 20 carbons and 4 double bonds beginning at the sixth carbon. As a result, it is referred to as "20 : 4 n-6". Similarly, docosahexaenoic acid (DHA) has a chain length of 22 carbons with 6 double bonds beginning with the third carbon from the methyl end and is thus designated "22 : 6 n-3". The biosynthetic pathways for AA (n-6 series) and DHA (n-3 series) from their respective C 18 precursors are distinct, but share elongation and desaturation steps and are well understood. Thus, other important LC-PUFAs are the C 18 fatty acids that are precursors in these biosynthetic pathways, for example, linoleic (18 : 2 n-6), a-linolenic (18 : 3 n-3) and stearidonic (18 : 4 n-3) in the n-3 pathway.

Numerous studies show that adequate intakes of omega 3 PUFAs are essential for the normal growth and development of infant brain. It is well established that omega 3 LC-PUFA reduce production of proinflammatory cytokines. On the other hand, an excess of omega 6 LC-PUFA in peripheral blood relative to levels of omega 3 fatty acids promotes overproduction of proinflammatory cytokines.

As structural components of membranes, AA and DHA influence the functional characteristics of certain tissues, particularly neural tissues. AA is the major precursor for the synthesis of eicosanoids, powerful cellular regulatory substances and mediators of inflammation that include prostaglandins, thromboxanes, and leukotrienes. Eicosanoids are involved in platelet aggregation, T-cell proliferation, lymphocyte migration, vasoconstriction and dilation, and the production of several immune and inflammatory substances. In the presence of eicosapentanoic acid (EPA), eicosanoids derived from the omega-3 family are also produced, and these attenuate or inhibit the action of the AA-derived eicosanoids. Those derived from EPA also have weaker biological activity than those from AA. Competition from omega-3 fatty acids for the same eicosanoid synthesizing enzymes may reduce the production of omega-6 eicosanoids. Thus, the presence of EPA can moderate the production and activity of AA-derived eicosanoids. It is believed that the predominance of eicosanoids derived from omega-6 fatty acids contributes to the development and deterioration of several chronic diseases, including heart disease.

According to a preferred embodiment of the invention, the infant formula additionally comprises one or more polyunsaturated long chain fatty acids (PUFA), including but not limited to linoleic acid, α-linolenic acid, oleic acid, arachidonic acid (AA) and docosahexaenoic acid (DHA). According to a preferred embodiment of the invention, the infant formula comprises up to 0.4% of docosahexaenoic acid and up to 0.6 % of arachidonic acid of the total of fatty acids present in the formula.

Another compound of special interest in the present invention, which is involved in the improvement of the regulation and stimulation of the development of the immune system is lactoferrin.

Lactoferrin is a glycoprotein that belongs to the iron transporter or transferrin family. It was originally isolated from bovine milk, where it is found as a minor protein component of whey proteins. Owing to its iron-binding properties, lactoferrin is thought to play a role in iron uptake by the intestinal mucosa of the suckling neonate. That is, it appears to be the source of iron for breast-fed infants. It also appears to have antibacterial, antiviral, antifungal, antiinflammatory, antioxidant and immunomodulatory activities.

The possible antibacterial activity of supplemental lactoferrin might be accounted for, in part, by its ability to strongly bind iron. Iron is essential to support the growth of pathogenic bacteria. Lactoferrin may also inhibit the attachment of bacteria to the intestinal wall. A breakdown product of lactoferrin is the peptide lactoferricin. Lactoferricin, classified as a bioactive peptide, may also have antibacterial, as well as antiviral, activity. The possible antiviral activity of supplemental lactoferrin may be due to its inhibition of virus-cell fusion and viral entry into cells.

The lactoferrin which is employed in the invention can be derived from any mammal; preferably, the lactoferrin is human or bovine in origin. Lactoferrin from other ruminants (e.g., sheep and goats) is also preferred. Nevertheless, other lactoferrins (e.g. produced with recombinant DNA techniques or produced in a prokaryotic cell) may also used herein.

According to a preferred embodiment of the invention, besides the above mentioned components, the composition of the invention contains lactoferrin. More preferred are those compositions which comprises up to 100 mg/100 ml of lactoferrin.

Besides the above-mentioned components, the composition of the invention may further contain any component or ingredient for infant formula known per se, in usual amounts (e.g. as prescribed by national or international guidelines) and depending upon (the properties of) the final formula desired. Such further components may include, but are not limited to, one or any combination of the following:
- one or more of the following compounds: taurine, choline, carnitine, inositol, biotin;
- one or more medium chain triglicerides ;
- one or more carbohydrates, including but not limited to lactose, dextrinomaltose, glucose, and/or galactose;
- one or more nucleotides and/or nucleotide analogues;
- one or more (added) amino acids, including but not limited to tryptophan and/or methionine;
- one or more (added) vitamins, including but not limited to vitamin K, vitamin B1, vitamin B2, vitamin A, vitamin E, vitamin D, vitamin C, niacin, pantothenic acid. Also, besides the usual amounts of these or other vitamins, the nutritional formula of the invention may contain additional amounts of - for instance - folic acid, vitamin B12 and/or vitamin B6;
- one or more probiotics, such as *Lactobacillus* or *Bifidobacteria* genera;
- one or more minerals and/or trace elements.

According to a more preferred embodiment, the amounts of the components will be generally as follows:
a) up to 1.37 g/100 ml of colostrum or a colostrum-derived product;
b) up to 1.2 g/100 ml of a prebiotic component selected from the group consisting of GOS, inulin and mixtures thereof;
   and optionally one or more of the following components:
c) up to 0.4% of DHA and up to 0.6 % of AA of the total of fatty acids present in the formula;
d) up to 100 mg/100 ml of lactoferrin.

More preferably, the nutritional composition and/or infant formula according to the present invention comprises:
a) up to 1.37 g/100 ml of colostrum or a colostrum-derived product;
b) up to 1.2 g/100 ml of a prebiotic component selected from the group consisting of galactooligosaccharides, inulin and mixtures thereof;
c) up to 0.4% of docosahexaenoic acid and up to 0.6 % of arachidonic acid of the total of fatty acids present in the formula;
and optionally d) up to 100 mg/100 ml of lactoferrin.

More preferred are those wherein the prebiotic component present in the infant formula comprises up to 1.2 g/100 ml of GOS.

Infant formulas of particular interest according to the present invention, are those which comprises 0.8 g/100 ml of GOS. Infant formulas comprising the above mentioned amount of GOS, are of particular interest intended for particular nutritional use by infants during the first four to six months of life, i.e. "pre-term" and "term" formulas.

Another infant formulas of particular interest according to the present invention, are those which comprises 0.44 g/100 ml of GOS. Infant formulas comprising the above mentioned amount of GOS, are of particular interest intended for particular nutritional use by infants aged over four months, i.e. "follow-on formulas".

The infant formula of the invention may generally be prepared by mixing/combining the different components/ingredients in the amounts mentioned herein. This may generally be carried out in a manner known per se using well known mixing and/or processing equipment. The infant formula may also be heated/sterilised (e.g. by UHT-treatment) and is then preferably brought into a powder form, e.g. by evaporation or spray-drying; and aseptically packaged, e. g. in a suitable container such as a tin, a box, a bag, a flask or a sachet. A preferred method for preparing the infant formula of the invention, will be discussed in more detail below.

Briefly, the processing method comprises pasteurizing the milk at 75 °C during 20 seconds, cooling down the milk and to skimming it. Then, the skimmed milk undergo a heating/sterilising treatment by UHT-treatment and is concentrated from 9% to 23% of dry extract. Then, the skimmed concentrated milk is filtered and heated until 70 °C; afterwards it is added to an appropriately sized tank with agitation and heating and the rest of ingredients are added. Then, the mixed ingredients are filtered and sterilised. The resulting product is homogenized and cooled down. The formula is concentrated until 50-52% of dry extract.

The daily effective dosage of the composition of this invention will depend upon the size of the individual (human or animal) being treated, the condition being treated, the age of the individual and other factors well known to the physician or veterinarian in attendance. The optimum daily dosage can easily be determined by a few simple observations. As the term is used herein, "effective dosage" means that dosage which will bring about the desired result, i.e. enhancement of the regulation and stimulation of the development of the immune system.

Throughout the description and claims the word "comprise" and variations of the word, such as "comprising", are not intended to exclude other technical features, additives, components, or steps.

The compositions may be prepared by conventional methods well known to the skilled artisan and may be provided in a wide variety of forms suitable for the selected method of administration. Therefore, the nutritional formula of the invention can have the form of liquid or powder, that can be reconstituted with water to produce a ready to feed formulation. It can also have the form of a meal that is used for wearing purposes or similar product evident to a person skilled in the art.

According to an embodiment of the invention, the compositions may be formulated as an infant formula for infants of less than 4-6 months old, or as a infant formula for infants of more than 4-6 months old.

Preferably, the infant formula of the invention, has an osmolarity between 200 to 400 mOsm/l. Preferred are those wherein the osmolarity is between 230 to 300 mOsm/l. Most preferred are those wherein the osmolarity is between 250 to 290 mOsm/l.

The energy value of the infant formula according to the present invention is preferably about 500 kcal/l to about 1500 kcal/I; more preferably from about 600 to about 800 kcal/l. Most preferred are those wherein the energy density of the infant formula is about 680 kcal/l to about 750 kcal/l.

Additional objects, advantages and features of the invention will become apparent to those skilled in the art upon examination of the description or may be learned by practice of the invention. The following examples are provided by way of illustration, and are not intended to be limiting of the present invention.

### DETAILED DESCRIPTION OF PARTICULAR EMBODIMENTS

### Example 1.- Preparation of an infant formula in powder form.

An infant formula, in the form of a term formula intended for particular nutritional use by infants during the first four to six months of life, in powder form was prepared using the following formula:

| INGREDIENTS | UNITS | 100 g POWDER |
|---|---|---|
| Whey protein | g | 6,6 |
| Casein | g | 4,4 |
| Colostrum | mg | 118,8 |
| Fats (enriched in LC-PUFAs) | g | 26,0 |
| Lactose | g | 36,5 |
| Dextrinomaltose | g | 13,5 |
| Glucose | g | 1,6 |
| Galactose | g | 0,9 |
| GOS | g | 5,7 |
| Coline | mg | 51,0 |
| Taurine | mg | 32,0 |
| Inositol | mg | 25,0 |
| L-Carnitine | mg | 17,0 |
| Sodium | mg | 175,0 |
| Potasium | mg | 535,0 |
| Chloride | mg | 290,0 |
| Calcium | mg | 420,0 |
| Phosphorus | mg | 230,0 |
| Iron | mg | 7,0 |
| Magnesium | mg | 42,0 |
| Zinc | mg | 7,0 |
| Copper | mcg | 300,0 |
| Iodine | mcg | 70,0 |
| Manganesium | mcg | 50,0 |
| Selenium | mcg | 14,0 |
| Vitamin A | mcg | 640,0 |
| Vitamin D | mcg | 10,3 |
| Vitamin E | mg | 25,0 |
| Vitamin K | mcg | 42,0 |
| Vitamin B1 | mcg | 520,0 |
| Vitamin B2 | mcg | 620,0 |
| Vitamin B6 | mcg | 825,0 |
| Vitamin B12 | mcg | 2,0 |
| Vitamin C | mg | 60,0 |
| Folic Acid | mcg | 60,0 |
| P. Calcium | mg | 3,2 |
| Nicotinamide | mg | 6,0 |
| Biotin | mcg | 16,0 |
| Citidine 5' monophosphate | mg | 7,9 |
| Uridine 5' monophosphate | mg | 5,3 |
| Adenosine 5' monophosphate | mg | 2,7 |
| Guanosine 5' monophosphate | mg | 1,6 |
| Inosine 5' monophosphate | mg | 1,6 |

### Example 2.- Preparation of follow-on infant formula in powder form.

An infant formula in powder form, intended for particular nutritional use by infants aged over four months was prepared using the following formula:

| INGREDIENTS | UNITS | 100 g POWDER |
|---|---|---|
| Whey protein | g | 6,1 |
| Casein | g | 6,1 |
| Colostrum | mg | 119,0 |
| Fats (enriched in LC-PUFAs) | g | 22,0 |
| Lactose | g | 35,35 |
| Dextrinomaltose | g | 22,00 |
| Glucose | g | 0,80 |
| Galactose | g | 0,05 |
| GOS | g | 3,0 |
| Coline | mg | 50,0 |
| Taurine | mg | 33,0 |
| Inositol | mg | 25,0 |
| L-Carnitine | mg | 16,0 |
| Sodium | mg | 290,0 |
| Potasium | mg | 780,0 |
| Chloride | mg | 555,0 |
| Calcium | mg | 615,0 |
| Phosphorus | mg | 415,0 |
| Iron | mg | 8,0 |
| Magnesium | mg | 60,0 |
| Zinc | mg | 10,0 |
| Copper | mcg | 450,0 |
| Iodine | mcg | 85,0 |
| Manganesium | mcg | 80,0 |
| Selenium | mcg | 14,0 |
| Vitamin A | mcg | 620,0 |
| Vitamin D | mcg | 12,0 |
| Vitamin E | mg | 24,0 |
| Vitamin K | mcg | 42,0 |
| Vitamin B1 | mcg | 550,0 |
| Vitamin B2 | mcg | 880,0 |
| Vitamin B6 | mcg | 820,0 |
| Vitamin B12 | mcg | 2,0 |
| Vitamin C | mg | 60,0 |
| Folic Acid | mcg | 105,0 |
| P. Calcium | mg | 3,2 |
| Nicotinamide | mg | 10,0 |
| Biotin | mcg | 16,0 |
| Citidine 5' monophosphate | mg | 7,8 |
| Uridine 5' monophosphate | mg | 5 |
| Adenosine 5' monophosphate | mg | 2,6 |
| Guanosine 5' monophosphate | mg | 1,5 |
| Inosine 5' monophosphate | mg | 1,5 |
| B. Infantis | ---- 1x10⁷ UFC/g of milk ----- | |
| L. Casei Rhamnosus | ---- 1x10⁷ UFC/g of milk ----- | |

### Example 3.- Preparation of an infant formula in powder form, supplemented with lactoferrin.

An infant formula, in the form of a term formula intended for particular nutritional use by infants during the first four to six months of life, in powder form was prepared using the following formula:

| INGREDIENTS | UNITS | 100 g POWDER |
|---|---|---|
| Whey protein | g | 6,35 |
| Casein | g | 4,4 |
| Colostrum | mg | 118,8 |
| Lactoferrin | mg | 350,0 |
| Fats (enriched in LC-PUFAs) | g | 26,0 |
| Lactose | g | 36,5 |
| Dextrinomaltose | g | 13,5 |
| Glucose | g | 1,6 |
| Galactose | g | 0,9 |
| GOS | g | 5,7 |
| Coline | mg | 51,0 |
| Taurine | mg | 32,0 |
| Inositol | mg | 25,0 |
| L-Carnitine | mg | 17,0 |
| Sodium | mg | 175,0 |
| Potasium | mg | 535,0 |
| Chloride | mg | 290,0 |
| Calcium | mg | 420,0 |
| Phosphorus | mg | 230,0 |
| Iron | mg | 7,0 |
| Magnesium | mg | 42,0 |
| Zinc | mg | 7,0 |
| Copper | mcg | 300,0 |
| Iodine | mcg | 70,0 |
| Manganesium | mcg | 50,0 |
| Selenium | mcg | 14,0 |
| Vitamin A | mcg | 640,0 |
| Vitamin D | mcg | 10,3 |
| Vitamin E | mg | 25,0 |
| Vitamin K | mcg | 42,0 |
| Vitamin B1 | mcg | 520,0 |
| Vitamin B2 | mcg | 620,0 |
| Vitamin B6 | mcg | 825,0 |
| Vitamin B12 | mcg | 2,0 |
| Vitamin C | mg | 60,0 |
| Folic Acid | mcg | 60,0 |
| P. Calcium | mg | 3,2 |
| Nicotinamide | mg | 6,0 |
| Biotin | mcg | 16,0 |
| Citidine 5' monophosphate | mg | 7,9 |
| Uridine 5' monophosphate | mg | 5,3 |
| Adenosine 5' monophosphate | mg | 2,7 |
| Guanosine 5' monophosphate | mg | 1,6 |
| Inosine 5' monophosphate | mg | 1,6 |

### Example 4.- Preparation of "follow-on formula" in powder form, supplemented with lactoferrin.

An infant formula in powder form, intended for particular nutritional use by infants aged over four months was prepared using the following formula:

| INGREDIENTS | UNITS | 100 g POWDER |
|---|---|---|
| Whey protein | g | 6,0 |
| Casein | g | 6,0 |
| Colostrum | mg | 119,0 |
| Lactoferrin | mg | 350,0 |
| Fats (enriched in LC-PUFAs) | g | 22,0 |
| Lactose | g | 35,35 |
| Dextrinomaltose | g | 22,00 |
| Glucose | g | 0,80 |
| Galactose | g | 0,05 |
| GOS | g | 3,0 |
| Coline | mg | 50,0 |
| Taurine | mg | 33,0 |
| Inositol | mg | 25,0 |
| L-Carnitine | mg | 16,0 |
| Sodium | mg | 290,0 |
| Potasium | mg | 780,0 |
| Chloride | mg | 555,0 |
| Calcium | mg | 615,0 |
| Phosphorus | mg | 415,0 |
| Iron | mg | 8,0 |
| Magnesium | mg | 60,0 |
| Zinc | mg | 10,0 |
| Copper | mcg | 450,0 |
| Iodine | mcg | 85,0 |
| Manganesium | mcg | 80,0 |
| Selenium | mcg | 14,0 |
| Vitamin A | mcg | 620,0 |
| Vitamin D | mcg | 12,0 |
| Vitamin E | mg | 24,0 |
| Vitamin K | mcg | 42,0 |
| Vitamin B1 | mcg | 550,0 |
| Vitamin B2 | mcg | 880,0 |
| Vitamin B6 | mcg | 820,0 |
| Vitamin B12 | mcg | 2,0 |
| Vitamin C | mg | 60,0 |
| Folic Acid | mcg | 105,0 |
| P. Calcium | mg | 3,2 |
| Nicotinamide | mg | 10,0 |
| Biotin | mcg | 16,0 |
| Citidine 5' monophosphate | mg | 7,8 |
| Uridine 5' monophosphate | mg | 5 |
| Adenosine 5' monophosphate | mg | 2,6 |
| Guanosine 5' monophosphate | mg | 1,5 |
| Inosine 5' monophosphate | mg | 1,5 |
| B. Infantis | ---- 1x10⁷ UFC/g of milk ---- | |
| L. Casei Rhamnosus | ---- 1x10⁷ UFC/g of milk ---- | |

### Example 5.- Study of the clinical recovery of term infants after infection events

The effects of the infant formula according to the present invention, over the clinical recovery of term infants after infection events was tested in a multi-center, prospective, randomized, controlled clinical trial involving 108 term infants of less that 12 month of age.

The infants were distributed randomly in 3 groups:
1. CONTROL (term infants fed with a common infant formula)
2. GOS (term infants fed with an infant formula supplemented with 0.8 g/100 ml GOS)
3. CAL-GOS (term infants fed with an infant formula supplemented with 0.8 g/100 ml GOS plus 16.6 mg/100 ml of a colostrum-derived product as defined in the present invention).

After prolonged feeding of the formulas, a reduction statistically significant in the time of presence of digestive symptoms associated to infection (such as diarrheas and vomits) was observed in the CAL-GOS group. Wherein the average duration of the diarrheic processes of 9 days in the CONTROL group, 4.8 in the GOS group and 3.2 days in the CAL-GOS group (p<0.05).

Furthermore, in the CAL-GOS group the time necessary to restore the appetite (p=0.0001) was less than the other groups (p=0.001), being of 11 days for the CONTROL group, 5.5 days for the GOS group and 3.9 days for the CAL-GOS group. Also the ponderal recuperation (p=0.0001) of the lactant was more early in the CAL-GOS group with regard to the other groups.

Therefore, the infant formula supplemented with colostrum and GOS according to the present invention demonstrated to present major efficiency to obtain a rapid and complete recovery of the lactant in a minor period of time with regard to the milk that contained only GOS and this one with regard to the milk control.

### Example 6.- Analysis of biochemical parameters in connection with the regulation and stimulation of the development of the immune system.

The effects of the formulas of example 1 and 2 are tested in a study involving term infants and is compared to the effects of a standard commercial product not containing colostrum or a colostrum-derived product as defined in the present invention and no containing GOS. After prolonged feeding of the formulas, several biochemical parameters in connection with the regulation and stimulation of the development of the immune system, are analyzed.

Biochemical determination of interleukins (such as TNF and IL-8), immunoglobulins (such as IgA₂, IgG, IgM), neopterin, essential fatty acids, macrophages, and lymphocytes is carried out in order to evaluate the incidence of the infant formulas in study over the increase and improvement of all examined immunity parameters. All these immunity parameters and indices have previously been shown to be associated with various effective functions of human immune system and general systemic body defense reactions.

The results show that all of the infants feed with the infant formula of the present invention have an improved profile in their immune system, as shows the biochemical parameters analyzed, as compared with the standard commercial product not containing colostrum or a colostrum-derived product as defined in the present invention and GOS.

## Claims

1. A nutritional composition which provides an improvement of the regulation and stimulation of the development of the immune system comprising colostrum or a colostrum-derived product and a prebiotic component selected from the group consisting of galactooligosaccharides, inulin and mixtures thereof.

2. The nutritional composition according to claim 1, which comprises up to 1.37 g/100 ml of colostrum or a colostrum-derived product.

3. The nutritional composition according to any of claims 1 to 2, wherein the colostrum-derived product comprises between 75-90 g /100 g of whey protein.

4. The nutritional composition according to any of claims 1 to 3, which comprises up to 1.2 g/100 ml of the prebiotic component.

5. The nutritional composition according to claim 4, which comprises up to 0.8 g/100 ml of the prebiotic component.

6. The nutritional composition according to any of claims 1 to 5, wherein the prebiotic component comprises at least one galactooligosaccharide.

7. The nutritional composition according to any of claims 1 to 5, wherein the prebiotic component comprises at least one inulin.

8. The nutritional composition according to claim 7, wherein the inulin consist of a mixture of an easily fermentable inulin component and a hardly fermentable inulin component in a specific weight ratio ranging from 10/90 to 70/30.

9. The nutritional composition according to any of claims 1 to 8, wherein the formula additionally comprises one or more polyunsaturated long chain fatty acids.

10. The nutritional composition according to claim 9, wherein the polyunsaturated long chain fatty acid comprises up to 0.4% of docosahexaenoic acid and up to 0.6 % of arachidonic acid of the total of fatty acids present in the formula.

11. The nutritional composition according to any of claims 1 to 10, wherein the formula additionally comprises lactoferrin.

12. The nutritional composition according to claim 11, which comprises up to 100 mg/100 ml of lactoferrin.

13. The nutritional composition according to any of claims 1 to 11, which comprises:
a) up to 1.37 g/100 ml of colostrum or a colostrum-derived product;
b) up to 1.2 g/100 ml of a prebiotic component selected from the group consisting of galactooligosaccharides, inulin and mixtures thereof;
c) up to 0.4% of docosahexaenoic acid and up to 0.6 % of arachidonic acid of the total of fatty acids present in the formula;
and optionally d) up to 100 mg/100 ml of lactoferrin.

14. The nutritional composition according to claim 13, which it further comprises probiotics, nucleotides, minerals, vitamins or mixtures thereof.

15. The nutritional composition according to any of claims 13 to 14, which comprises up to 1.2 g/100 ml of galactooligoosaccharides.

16. The nutritional composition according to any of claims 13 to 15, which comprises up to 100 mg/100 ml of lactoferrin.

17. An infant formula which provides an improvement of the regulation and stimulation of the development of the immune system which comprises the nutritional composition according to any of claims 1 to 16.

18. Use of an infant formula according to claim 17 to improve the regulation and stimulation of the development of the immune system.
